# EUROPEAN PATENT APPLICATION

(11) **EP 4 012 410 A1**
(43) Date of publication of application: **15.06.2022**
(21) Application number: 20874014.2
(22) Date of filing: 10.10.2020
(51) Int. Cl.: G01N 33/543, G01N 33/532

(54) **MAGNETIC PARTICLE LUMINESCENCE MICRO-FLUIDIC CHIP FOR MULTI-MARKER DETECTION, AND DETECTION DEVICE**

(30) Priority: 11.10.2019 CN 201910962433
(71) Applicant: Shenzhen Watmind Medical Co., Ltd., Shenzhen Guangdong 518000 (CN)
(72) Inventor: WANG, Dong, Shenzhen, Guangdong 518000 (CN); JIANG, Rongxiang, Shenzhen, Guangdong 518000 (CN); LI, Quan, Shenzhen, Guangdong 518000 (CN)
(74) Representative: Petraz, Gilberto Luigi
(86) International application number: PCT/CN2020/120090
(87) International publication number: WO 2021/068913

(57) **Abstract**

The present invention relates to the field of luminescence immunoassay technologies for micro-fluidic chips, and in particular, to a magnetic particle luminescent micro-fluidic chip for multi-marker detection and a detection apparatus. The chip includes a top plate, provided with at least one sample loading part, a mixing area communicated with the sample loading part, and a plurality of labeling ligands arranged in the mixing area; a bottom plate, including a flow guide area communicated with the mixing area, reaction areas communicated with the flow guide area, a plurality of detection areas communicated with the reaction areas, as well as a cleaning liquid storage part and a luminescent liquid storage part which are communicated with the detection areas, where the reaction areas are provided with a plurality of magnetic particle ligands, and magnetic particles and ligands of the magnetic particle ligands are different; a cleaning liquid is stored in the cleaning liquid storage part, and a luminescent liquid is stored in the luminescent liquid storage part; and an air pump, disposed on the top plate and configured to drive a sample in the sample loading part to flow through the mixing area. Because the plurality of detection areas are arranged, cross interference between the magnetic particles can be avoided, and thus the accuracy of a detection result is greatly improved.

## Description

### TECHNICAL FIELD

The present invention relates to the field of luminescence immunoassay technologies for micro-fluidic chips, and in particular, to a magnetic particle luminescent micro-fluidic chip for multi-marker detection and a detection apparatus.

### BACKGROUND

There are currently two main trends in in vitro diagnostics (IVD): one is automation and integration, that is, high-precision disease analysis and diagnosis are achieved by using fully automated and highly sensitive large-scale instruments and devices in central laboratories of large hospitals; and the other is miniaturization and bedside, that is, rapid on-site analysis and diagnosis are achieved by small and simple handheld devices. However, it is unsuitable for small hospitals with insufficient funds and small sample size to purchase expensive large-scale devices. Therefore, rapid detection devices used by most hospitals at present are mainly test strips and associated devices thereof, but test strips can only achieve qualitative or semi-quantitative detection, has low detection sensitivity and poor specificity and repeatability, and is subjected to interference obviously. Due to a large population, aggravated aging, and a dramatic increase in incidence of diseases in China, it is overwhelmed by simply relying on large hospitals. Therefore, it is extremely urgent to develop rapid detection methods and devices with ease of operation, high sensitivity, good repeatability and accurate quantification.

Chemiluminescence refers to a phenomenon that chemical energy is converted into light energy by a reaction intermediate, a reaction product or an additive luminescent reagent during a chemical reaction. Compared with fluorescence and absorbed light, chemiluminescence is subjected no interference from a background signal of an external excitation light source and little cross interference, and has the advantages of high sensitivity, wide linear range, and the like. Chemiluminescence analysis established on this basis has been widely applied to such fields as clinical diagnostics. A chemiluminescence analyzer is a main large-scale IVD analysis and detection device.

A micro-fluidic chip technology integrates basic operating units for sample preparation, reaction, separation, detection, and the like in biological, chemical and medical analysis processes into a micron-scale chip to automatically complete a whole analysis process. Because of its great potential in the fields of biology, chemistry and medicine, the micro-fluidic chip technology has developed into an interdisciplinary research field of biology, chemistry, medicine, fluidics, materialogy, machinery, and the like, and has been applied to the fields of biomedical research, biochemical detection, forensic authentication, and the like.

Only one detection area is provided for an existing micro-fluidic chip. When different labeling ligands are required for a sample, the sample is mixed with the labeling ligands and then enters a reaction area to react with magnetic particle ligands. After the reaction, the sample enters the detection area for quantitative sample analysis and detection. However, when the sample enters the detection area, an external magnet is needed to drive magnetic particles of the magnetic particle ligands to move to the detection area. Because of different volumes, weights and nucleo-cytoplasmic ratios of different magnetic particles, the magnet has different aggregation and attraction effects on different magnetic particles, and magnetic particles with a larger volume, weight and nucleo-cytoplasmic ratio move faster than those with a smaller volume, weight and nucleo-cytoplasmic ratio to allow the magnetic particles to enter the detection area sequentially by using different moving speeds of the magnetic particles. Because only one detection area is arranged, all magnetic particles enter the detection area. Although the magnetic particles move at different speeds, the magnetic particles have a shorter stroke especially when the magnet is moving faster, which still easily causes cross interference between the magnetic particles, thereby greatly reducing the accuracy of a detection result.

### SUMMARY

Embodiments of the present invention provide a magnetic particle luminescent micro-fluidic chip for multi-marker detection and a detection apparatus, aiming to resolve problems that magnetic particles are prone to cross interference, and the accuracy of a detection result is greatly reduced when an existing magnetic particle luminescent micro-fluidic chip is used for detection.

The embodiments of the present invention are implemented as follows: A magnetic particle luminescent micro-fluidic chip for multi-marker detection is provided. The chip includes a top plate, provided with at least one sample loading part, a mixing area communicated with the sample loading part, and a plurality of labeling ligands arranged in the mixing area, the labeling ligands being different from each other; a bottom plate, including a flow guide area communicated with the mixing area, reaction areas communicated with the flow guide area, a plurality of detection areas communicated with the reaction areas, as well as a cleaning liquid storage part and a luminescent liquid storage part which are communicated with the detection areas, where the reaction areas are provided with a plurality of magnetic particle ligands, the magnetic particle ligands include magnetic particles and ligands, and the magnetic particles and the ligands of the magnetic particle ligands are different; a cleaning liquid is stored in the cleaning liquid storage part, and a luminescent liquid is stored in the luminescent liquid storage part; and an air pump, disposed on the top plate and configured to drive a sample in the sample loading part to flow through the mixing area.

Further, at least one of a nucleo-cytoplasmic ratio, mass and a volume of the magnetic particles is different.

Further, both sides of a channel in the detection areas are provided with a positive electrode and a negative electrode respectively, and the magnetic particles have different isoelectric points.

Further, the mixing area is provided with a labeling ligand storage part, and labeling ligands are stored in the labeling ligand storage part.

Further, the number of the sample loading parts is consistent with that of the labeling ligand storage parts.

Further, the sample loading part includes a sample loading port and a cover for opening or closing the sample loading port, and the sample loading part further includes a rubber ring disposed on the sample loading port.

Further, the flow guide area is internally provided with a groove with a height less than those of bottom walls of the reaction areas, and a flow guide part arranged on the groove and connected to the reaction areas.

Further, the detection areas include at least one cleaning area and a plurality of luminescent areas which are communicated with each other.

Further, the number of the cleaning areas is consistent with that of the luminescent areas, and the cleaning areas are arranged corresponding to the luminescent areas.

The present invention further provides a magnetic particle luminescent micro-fluidic detection apparatus for multi-marker detection. The detection apparatus includes the magnetic particle luminescent micro-fluidic chip as described above; a magnet unit for driving the magnetic particles to move; a squeezing unit for squeezing the cleaning liquid storage part, the luminescent liquid storage part and the air pump; and a detection unit for detecting a luminescence signal in the detection areas.

Compared with the prior art, the present invention has the following beneficial effects: The present invention provides a magnetic particle luminescent micro-fluidic chip for multi-marker detection and a detection apparatus. A sample is loaded from a sample loading part of a top plate and mixes with labeling ligands in a mixing area, then enters a flow guide area, enters reaction areas of a bottom plate from the flow guide area, and enters different detection areas from the reaction areas. In this case, an external magnet drives magnetic particles of magnetic particle ligands into different detection areas for luminescence detection in the detection areas. Because a plurality of detection areas are arranged, cross interference between the magnetic particles can be avoided, and thus the accuracy of a detection result is greatly improved.

### BRIEF DESCRIPTION OF DRAWINGS

FIG. 1 is a schematic diagram of a magnetic particle luminescent micro-fluidic chip provided with a plurality of labeling ligand storage parts and luminescent areas according to an embodiment of the present invention;
FIG. 2 is a schematic diagram of a magnetic particle luminescent micro-fluidic chip provided with a plurality of sample loading parts, labeling ligand storage parts and luminescent areas according to an embodiment of the present invention;
FIG. 3 is a schematic diagram of a magnetic particle luminescent micro-fluidic chip provided with a plurality of labeling ligand storage parts, cleaning areas and luminescent areas according to an embodiment of the present invention; and
FIG. 4 is a schematic diagram of a magnetic particle luminescent micro-fluidic chip provided with a plurality of sample loading parts, labeling ligand storage parts, cleaning areas and luminescent areas according to an embodiment of the present invention.

### DESCRIPTION OF EMBODIMENTS

To make the objectives, technical solutions, and advantages of the present invention clearer, the present invention will be further described in detail below with reference to the accompanying drawings and embodiments. It should be understood that specific embodiments described herein are merely intended to explain the present invention, and are not intended to limit the present invention.

The present invention provides a magnetic particle luminescent micro-fluidic chip for multi-marker detection and a detection apparatus. A sample is loaded from a sample loading part 11 on a top plate 1 and mixes with labeling ligands in a mixing area 12, then enters a flow guide area 21, enters reaction areas 22 of a bottom plate 2 from the flow guide area 21, and enters different detection areas from the reaction areas 22. In this case, an external magnet drives magnetic particles of magnetic particle ligands into different detection areas for luminescence detection in the detection areas. Because a plurality of detection areas are arranged, cross interference between the magnetic particles can be avoided, and thus the accuracy of a detection result is greatly improved.

### Embodiment 1

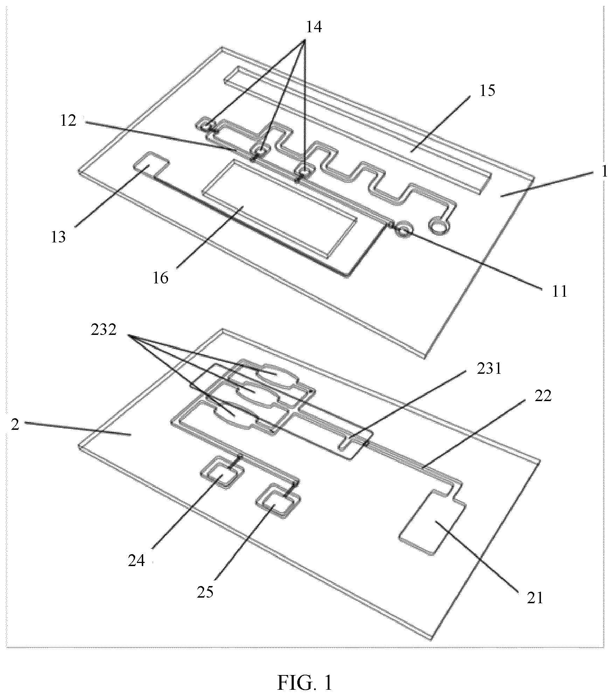

Referring to FIG. 1 to FIG. 4, this embodiment provides a magnetic particle luminescent micro-fluidic chip for multi-marker detection. The chip includes a top plate 1, provided with at least one sample loading part 11, a mixing area 12 communicated with the sample loading part 11, and a plurality of labeling ligands arranged in the mixing area 12, the labeling ligands being different from each other; a bottom plate 2 arranged on the top plate 1, including a flow guide area 21 communicated with the mixing area 12, reaction areas 22 communicated with the flow guide area 21, a plurality of detection areas communicated with the reaction areas 22, as well as a cleaning liquid storage part 24 and a luminescent liquid storage part 25 which are communicated with the detection areas, where the reaction areas 22 are provided with a plurality of magnetic particle ligands, the magnetic particle ligands include magnetic particles and ligands, and the magnetic particles and the ligands of the magnetic particle ligands are different; a cleaning liquid is stored in the cleaning liquid storage part 24, and a luminescent liquid is stored in the luminescent liquid storage part 25; and an air pump 13, disposed on the top plate 1 and configured to drive a sample in the sample loading part 11 to flow through the mixing area 12, where the air pump 13 may be an air bag built into the top plate 1.

For example, if the sample is a whole blood sample, the sample to be tested may be put into the sample loading part 11, and the air pump 13 is squeezed, then the sample enters the mixing area 12 through the sample loading part 11 to mix with a plurality of labeling ligands in the mixing area 12. After mixing, the sample enters the flow guide area 21 from the mixing area 12 that is provided with a blood filter membrane. Plasma in the sample is separated from blood cells, the plasma enters the reaction areas 22 from the flow guide area 21 and mixes with the magnetic particle ligands in the reaction areas 22, while the blood cells remain in the flow guide area 21.

After mixing with the magnetic particle ligands, the sample enters the detection areas from the reaction areas 22. In this case, an external magnet drives magnetic particles of the magnetic particle ligands into the detection areas from the reaction areas 22. Different magnetic particles can be driven to move by controlling a moving speed of the magnet, so that different magnetic particles can also enter different detection areas. Then, the cleaning liquid storage part 24 releases the cleaning liquid stored therein, and the cleaning liquid enters the detection areas to clean the magnetic particles. Then, the luminescent liquid storage part 25 releases the luminescent liquid stored therein, and the luminescent liquid enters the detection areas for quantitative detection of analytes in the sample. Because a plurality of different labeling ligands are provided, the labeling ligands can be mixed with the sample. After mixing, the sample enters the reaction areas 22 and eventually enters different detection areas. The sample that is finally analyzed and detected in the detection areas will not affect each other; and driven by the magnet, different magnetic particles enter different detection areas without cross interference between the magnetic particles, thereby greatly improving the accuracy of a detection result.

The blood filter membrane is formed in the flow guide area 21 in advance. The blood filter membrane can separate a liquid from cells by using physical apertures or a biological/chemical reagent to achieve separation of plasma from red blood cells, with the plasma flowing to the reaction areas 22 and the red blood cells remaining on the blood filter membrane, thereby reducing interference of the red blood cells to test results. The biological/chemical reagent includes a coagulant and the like, which can connect red blood cells to form clots and increase a size thereof, and red blood cells with an increased size are more likely to be blocked by a reticular structure of the blood filter membrane, thereby reducing interference of the red blood cells to the test results more effectively.

The cleaning liquid is pre-stored in the cleaning liquid storage part 24, and is used to clean magnetic particles to remove non-specific adsorbed analytes, luminescent agent markers and other substances that affect a detection result. The cleaning liquid mainly contains a buffer agent, a protein and a surfactant, where the buffer agent includes, but is not limited to, borate, phosphate, Tris-HCl, acetate, and the like. The cleaning liquid has a pH range of 6.0-10.0. The protein includes, but is not limited to, bovine serum albumin, casein, and the like. The surfactant includes, but is not limited to, Tween 20, Tween 80, Triton X-100, polyethylene glycol, polyvinylpyrrolidone, and the like. Preferably, in this embodiment, the cleaning liquid used is a pH7.0 Tris-HCl buffer containing bovine serum albumin, Tween 20 and Proclin 300.

The luminescent liquid is pre-stored in the luminescent liquid storage part 25, and is used to further clean the magnetic particles or enhance luminescence signals. The luminescent liquid contains a substrate liquid and a luminescent enhancement liquid, the substrate liquid may be an acidic solution containing luminol or an acidic solution containing adamantane, and the luminescent enhancement liquid may be an alkaline solution containing benzene derivatives.

It should be noted that, considering that the substrate liquid and the luminescent enhancement liquid should not be mixed and kept for a long time, the luminescent liquid storage part 25 may be provided with a first luminescent liquid storage part 25 and a second luminescent liquid storage part 25, the substrate liquid is stored in the first luminescent liquid storage part 25, the luminescent enhancement liquid is stored in the second luminescent liquid storage part 25, and a luminescent liquid mixing area 12 is provided on the bottom plate 2. The luminescent liquid mixing area 12 is communicated with the detection areas, the first luminescent liquid storage part 25 and the second luminescent liquid storage part 25. When released from the first luminescent liquid storage part 25 and the second luminescent liquid storage part 25, the substrate liquid and the luminescent enhancement liquid enter the luminescent liquid mixing area 12 and mix with each other, and then enter the detection areas after mixing well.

In this embodiment, the cleaning liquid storage part 24 and the luminescent liquid storage part 25 are sealed cavities, and a sealing material used is an elastic material or a high barrier film, which is specifically glass, plastic, rubber, aluminum foil or a high barrier film. The sealing material may be made of the same material, or may be made of multiple materials. Under physical squeezing, the cleaning liquid storage part 24 and the luminescent liquid storage part 25 may be partially broken to release materials stored therein.

### Embodiment 2

On the basis of Embodiment 1, in Embodiment 2, at least one of a nucleo-cytoplasmic ratio, mass and a volume of the magnetic particles is different. The magnetic particle ligands are placed in the reaction areas 22, and magnetic particles can be driven to move as the external magnet moves. By controlling a moving speed of the magnet, magnetic particles with a larger nucleo-cytoplasmic ratio, mass or volume are driven to move when the magnet moves faster; and magnetic particles with a smaller nucleo-cytoplasmic ratio, mass or volume are driven to move when the magnet moves slower. Therefore, a moving speed of the magnet can be controlled to drive magnetic particles that match the moving speed to move. Even though other magnetic particles will also move, only the magnetic particles that match the moving speed of the magnet can enter the detection areas through a channel between the reaction areas 22 and the detection areas. Therefore, the magnetic particles can be driven into the detection areas by controlling the moving speed of the magnet, and the magnetic particles in the detection areas will not interfere with each other.

### Embodiment 3

On the basis of Embodiment 1, in Embodiment 3, both sides of a channel in the detection areas are provided with a positive electrode and a negative electrode respectively. FIG. 1 shows locations of the positive electrode and the negative electrode. A "+" symbol as shown in FIG. 1 is the location of the positive electrode, and a "-" symbol as shown in FIG. 1 is the location of the negative electrode. Certainly, in other embodiments, the positive electrode and the negative electrode may alternatively be located in other locations, and details are not described herein again. The magnetic particles have different isoelectric points. An isoelectric point refers to a pH value when surfaces of magnetic particles are uncharged. The magnetic particle ligands are placed in the reaction areas 22, and magnetic particles can be driven to move as the external magnet moves. The magnet drives the magnetic particles to pass through the detection areas from the reaction areas 22, and then the magnet is withdrawn. Due to the positive electrode and the negative electrode on both sides of the channel in the detection areas, an electric field is formed, magnetic particles with more negative charges on surfaces will be attracted by the positive electrode, and magnetic particles with more positive charges on surfaces will be attracted by the negative electrode. Then the magnetic particles are moved into the detection areas by controlling the magnet to move. In this way, the magnetic particles can be moved into different detection areas only by matching the magnet with the electric field, without controlling the moving speed of the magnet.

### Embodiment 4

Referring to FIG. 1 to FIG. 4, on the basis of Embodiment 1, in Embodiment 4, the mixing area 12 is provided with a labeling ligand storage part 14, and labeling ligands are stored in the labeling ligand storage part 14. The labeling ligands are pre-stored in the labeling ligand storage part 14, so as to facilitate long-term storage of the labeling ligands and prevent deterioration and damage of the labeling ligands. In addition, different labeling ligands may be stored in different labeling ligand storage parts 14, so that enzyme labeling ratios required for different test items can be controlled, and different labeling ligands can be further prevented from affecting each other during storage.

When the labeling ligands include an enzyme-labeled ligand, the enzyme may be one or more of horseradish peroxidase and alkaline phosphatase, and the ligand may be one or more of an antigen, an antibody, hapten and a nucleic acid. A magnetic particle ligand solution is pre-stored in the reaction areas 22, and the magnetic particle ligand solution includes magnetic particles, saccharides, a buffer agent, a protein, a surfactant and a preservative. The magnetic particles include, but are not limited to, ferric oxide and ferroferric oxide compounds.

When the labeling ligands include an enzyme-labeled ligand, the enzyme binds to or competes with analytes in the sample to form an enzyme-labeled ligand; magnetic particle labels bind to or compete with the analytes in the sample to form a magnetic particle labeled ligand, and the two ligands may be the same or different. The magnetic labeled ligand and the enzyme-labeled ligand include nucleic acids, antigens, monoclonal antibodies, polyclonal antibodies and hormone receptors. The analytes in the sample include DNA, small molecules (medicines or drugs), antigens, antibodies, hormones, antibiotics, bacteria or viruses and other biochemical markers.

In this embodiment, the labeling ligands may bind to a magnetic particle ligand solution (for example, by double antibody sandwich ELISA), and the labeling ligands may compete with a labeling ligand (for example, by competitive ELISA). The enzyme-labeled ligand may be the same as or different from the magnetic particle ligand solution. Preferably, in one embodiment of the present invention, two different antibodies are selected as a labeling ligand and a magnetic particle ligand solution respectively to detect analytes by double antibody sandwich ELISA. In another embodiment of the present invention, an antigen and an antibody are selected as a labeling ligand and a magnetic particle ligand solution respectively to detect analytes by competitive ELISA.

### Embodiment 5

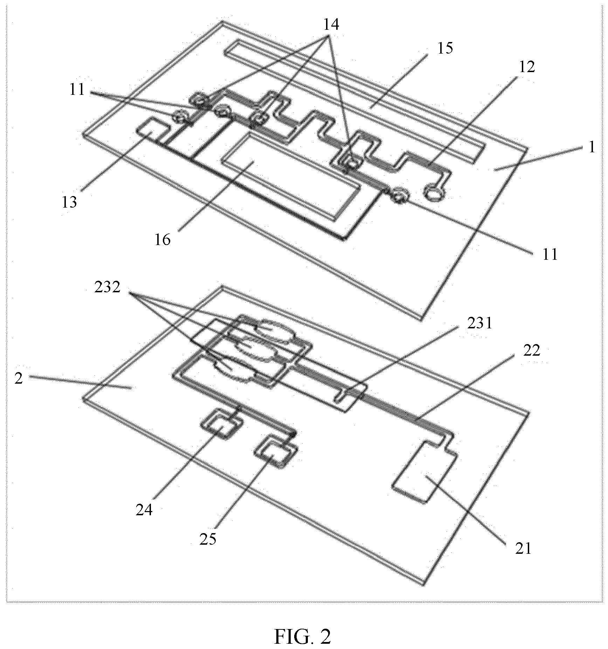

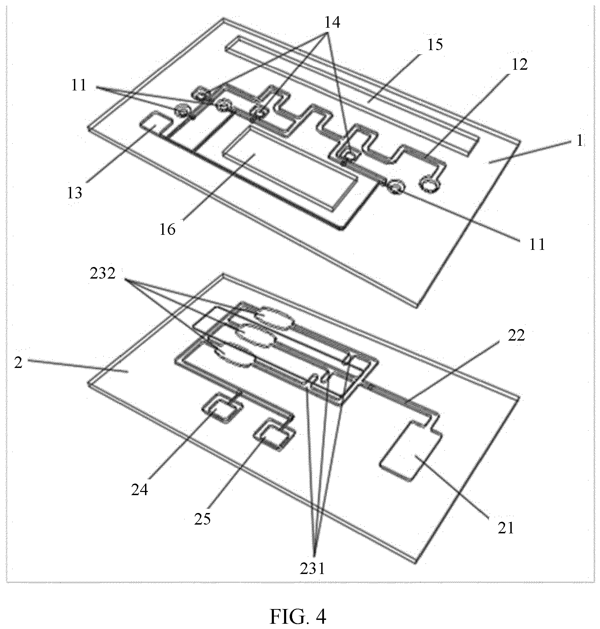

Referring to FIG. 2 and FIG. 4, on the basis of Embodiment 4, in Embodiment 5, the number of the sample loading parts 11 is consistent with that of the labeling ligand storage parts 14, and the sample loading parts 11 and the labeling ligand storage parts 14 are arranged in a one-to-one correspondence. In this way, both enzyme labeling ratios required for different test items and required samples can be controlled.

### Embodiment 6

On the basis of Embodiment 1, in Embodiment 6, the sample loading part 11 includes a sample loading port and a cover for opening or closing the sample loading port.

When the cover is open, a sample can be loaded from the sample loading port externally, and after the sample is loaded, the cover is closed to close the sample loading port.

Specifically, the cover is provided with a first clamping piece or a first clamping hole, and a second clamping hole or a second clamping piece is arranged at a location adjacent to the sample loading port to enable the cover to close the sample loading port through mutual matching between the first clamping piece and the second clamping hole or mutual matching between the first clamping hole and the second clamping piece. Moreover, the cover is further provided with a sealing member adapted to the sample loading port, and the sealing member is inserted into the sample loading port when the cover is closed to prevent the sample from leaking from the sample loading port.

Furthermore, the sample loading part 11 further includes a rubber ring arranged on the sample loading port. Because samples are often externally loaded through a pipette tip, the elastic rubber ring helps to seal with the pipette tips to inject the samples from the sample loading port more smoothly.

### Embodiment 7

On the basis of Embodiment 1, in Embodiment 7, the flow guide area 21 is internally provided with a groove with a height less than those of bottom walls of the reaction areas 22, and a flow guide part arranged on the groove and connected to the reaction areas 22. The flow guide part may be a blood filter membrane. Because the groove in the flow guide area 21 is lower than the bottom walls of the reaction areas 22, and similarly, the flow guide part has a height less than those of the bottom walls of the reaction areas 22, a sample will not automatically enter the reaction areas 22 from the flow guide part due to gravity after entering the flow guide area 21, but will be sucked away from the flow guide part by capillary action, so that a smaller volume of sample capable of meeting detection requirements can be sucked away from a larger volume of sample to prevent a larger sample size from affecting the detection result.

### Embodiment 8

Referring to FIG. 1 to FIG. 4, on the basis of Embodiment 1 to Embodiment 7, in Embodiment 8, the detection areas include at least one cleaning area 231 and a plurality of luminescent areas 232 which are communicated with each other. The flow guide area 21, the reaction areas 22, the cleaning areas 231 and the luminescent areas 232 are sequentially communicated with each other. After entering the reaction areas 22 from the flow guide area 21, the sample mixes with the magnetic particle ligands in the reaction areas 22, then enters the cleaning areas 231 from the reaction areas 22, and finally enters the detection areas from the cleaning areas 231. Meanwhile, the external magnet drives magnetic particles of the magnetic particle ligands into the cleaning areas 231 from the reaction areas 22. In this case, the cleaning liquid storage part 24 releases the cleaning liquid stored therein, and the cleaning liquid flows into the cleaning areas 231 to clean the magnetic particles, and then, different magnetic particles enter different detection areas from the cleaning areas 231. The sample can then be analyzed and detected in the detection areas.

### Embodiment 9

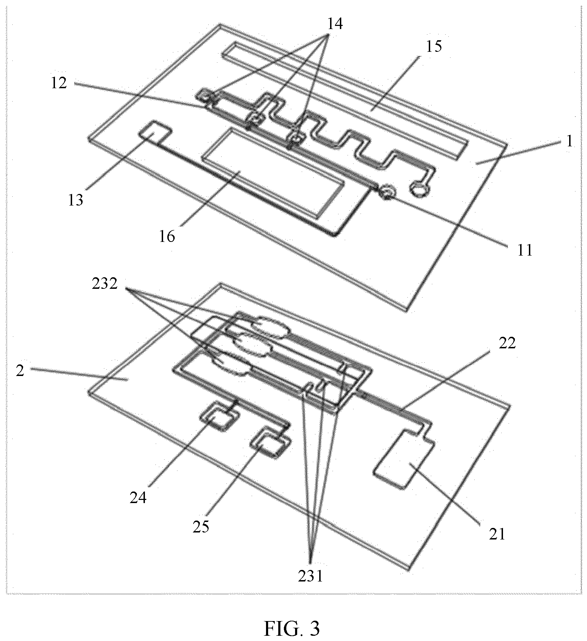

Referring to FIG. 3 and FIG. 4, on the basis of Embodiment 8, in Embodiment 9, the number of the cleaning areas 231 is consistent with that of the luminescent areas 232, and the cleaning areas 231 and the luminescent areas 232 are arranged in a one-to-one correspondence. If only one cleaning area 231 is arranged, there is a risk of cross interference when the magnetic particles are cleaned together. Therefore, a plurality of cleaning areas 231 are arranged, and the cleaning areas 231 are arranged corresponding to the luminescent areas 232, so that the magnetic particles can enter the corresponding luminescent areas 232 after cleaning, thereby further improving the accuracy of the detection result.

### Embodiment 10

Embodiment 10 provides a magnetic particle luminescent micro-fluidic detection apparatus for multi-marker detection. The detection apparatus includes the magnetic particle luminescent micro-fluidic chip according to Embodiment 1 to Embodiment 9; a magnet unit for driving the magnetic particles to move; a squeezing unit for squeezing the cleaning liquid storage part 24, the luminescent liquid storage part 25 and the air pump 13; and a detection unit for detecting a luminescence signal in the detection areas.

The magnet unit includes a magnet and a drive part for driving the magnet to move. The drive part may be a linear motor, and an output shaft of the linear motor is fixedly connected to the magnet. After the linear motor is started, the output shaft thereof extends out to drive the magnet to move, and the magnet attracts the magnetic particles and drives the magnetic particles to move.

The squeezing unit may be a linear motor. After the linear motor is started, an output shaft thereof extends out to crush the cleaning liquid storage part 24 and the luminescent liquid storage part 25 to allow the cleaning liquid and the luminescent liquid to flow out respectively. After the linear motor is started, the output shaft with extension and retraction reciprocating motion can repeatedly squeeze the air pump 13 to drive liquid on the top plate 1 to flow. Certainly, the output shaft of the linear motor may alternatively be fixedly provided with a squeezing part, the output shaft of the linear motor drives the squeezing part to move, and the squeezing part then squeezes the cleaning liquid storage part 24, the luminescent liquid storage part 25 and the air pump 13.

The detection unit may be a photodiode, a photomultiplier or an avalanche photodiode. Samples enter the detection areas after mixing and reaction through the above process, and the mixed samples will have a luminescence signal. The detection unit acquires the luminescence signal to obtain a detection result of the samples based on intensity of the luminescence signal.

When the magnetic particle luminescent double-layer micro-fluidic chip is provided with the labeling ligand storage part 14, the squeezing unit may also be used to crush the labeling ligand storage part 14 to allow the labeling ligands to flow out.

The magnetic particle ligands may be coded to accurately determine that the magnetic particles in different magnetic particle ligands enter different detection areas.

The foregoing descriptions are merely preferred embodiments of the present invention and are not intended to limit the present invention. Any modification, equivalent replacement, improvement, and the like made within the spirit and principle of the present invention shall fall within the protection scope of the present invention.

## Claims

1. A magnetic particle luminescent micro-fluidic chip for multi-marker detection, comprising:
a top plate, provided with at least one sample loading part, a mixing area communicated with the sample loading part, and a plurality of labeling ligands arranged in the mixing area, the labeling ligands being different from each other;
a bottom plate, comprising a flow guide area communicated with the mixing area, reaction areas communicated with the flow guide area, a plurality of detection areas communicated with the reaction areas, as well as a cleaning liquid storage part and a luminescent liquid storage part which are communicated with the detection areas, wherein
the reaction areas are provided with a plurality of magnetic particle ligands, the magnetic particle ligands comprise magnetic particles and ligands, and the magnetic particles and the ligands of the magnetic particle ligands are different;
a cleaning liquid is stored in the cleaning liquid storage part, and a luminescent liquid is stored in the luminescent liquid storage part; and
an air pump, disposed on the top plate and configured to drive a sample in the sample loading part to flow through the mixing area.

2. The magnetic particle luminescent micro-fluidic chip according to claim 1, wherein at least one of a nucleo-cytoplasmic ratio, mass and a volume of the magnetic particles is different.

3. The magnetic particle luminescent micro-fluidic chip according to claim 1, wherein both sides of a channel in the detection areas are provided with a positive electrode and a negative electrode respectively, and the magnetic particles have different isoelectric points.

4. The magnetic particle luminescent micro-fluidic chip according to claim 1, wherein the mixing area is provided with a labeling ligand storage part, and labeling ligands are stored in the labeling ligand storage part.

5. The magnetic particle luminescent micro-fluidic chip according to claim 4, wherein the number of the sample loading parts is consistent with that of the labeling ligand storage parts.

6. The magnetic particle luminescent micro-fluidic chip according to claim 1, wherein the sample loading part comprises a sample loading port and a cover for opening or closing the sample loading port, and the sample loading part further comprises a rubber ring disposed on the sample loading port.

7. The magnetic particle luminescent micro-fluidic chip according to claim 1, wherein the flow guide area is internally provided with a groove with a height less than those of bottom walls of the reaction areas, and a flow guide part arranged on the groove and connected to the reaction areas.

8. The magnetic particle luminescent micro-fluidic chip according to any one of claims 1 to 7, wherein the detection areas comprise at least one cleaning area and a plurality of luminescent areas which are communicated with each other.

9. The magnetic particle luminescent micro-fluidic chip according to claim 8, wherein the number of the cleaning areas is consistent with that of the luminescent areas, and the cleaning areas are arranged corresponding to the luminescent areas.

10. A magnetic particle luminescent micro-fluidic detection apparatus for multi-marker detection, comprising:
the magnetic particle luminescent micro-fluidic chip according to any one of claims 1 to 9;
a magnet unit for driving the magnetic particles to move;
a squeezing unit for squeezing the cleaning liquid storage part, the luminescent liquid storage part and the air pump; and
a detection unit for detecting a luminescence signal in the detection areas.
